# EUROPEAN PATENT APPLICATION

(11) **EP 1 321 434 A1**
(43) Date of publication of application: **25.06.2003**
(21) Application number: 01984564.3
(22) Date of filing: 30.08.2001
(51) Int. Cl.: C02F 1/68, A61K 7/00, A61K 33/44, C01B 31/08, B01J 13/00

(54) **METHOD AND APPARATUS FOR PREPARING AQUEOUS DISPERSION OF ULTRA-FINE ACTIVE CARBON PARTICLES**

(30) Priority: 31.08.2000 JP 2000262129
(71) Applicant: Phild Co., Ltd., Kyoto-city, Kyoto 602-0023 (JP)
(72) Inventor: HIRATA, Yoshihiro, Kamigyo-ku, Kyoto City, Kyoto 602-0023 (JP); UEDA, Yoshio, Kamigyo-ku,Kyoto City, Kyoto 602-0023 (JP); TAKASE, Hiroaki, Kamigyo-ku, Kyoto City, Kyoto 602-0023 (JP)
(74) Representative: VOSSIUS & PARTNER
(86) International application number: JP0107475
(87) International publication number: WO02018278

(57) **Abstract**

The purpose of the present invention is to provide a method and apparatus for producing high-function water containing ultra-fine active carbon particles, and which can be used for bioactivity improvement purposes. The method and apparatus given by the present invention facilitates the production of dispersed active carbon water in which ultra-fine active carbon particles are dispersed, by dispersing active carbon powder in high-pressure water and then combusting a gaseous mixture of oxygen and hydrogen in the high-pressure water to melt the active carbon powder using the resultant combustion gas.

## Description

### Field of the Invention

This invention relates to the method and system for producing bioactive water in which ultra-fine active carbon particles are dispersed, as well as the bioactive water applying the aforesaid method and system.

The invention also relates to health products, medical products and cosmetics as characterized by the utilization of the aforementioned bioactive water.

### Background of the Invention

Active carbon is a fine, black powder. Although the handling of this powder itself is rather troublesome, active carbon plays an essential part in daily life. The key applications of active carbon powder include water purification and wastewater treatment in industrial plants and breweries, while those of active carbon granules encompass gas and water treatments. The scope of application of active carbon is practically limitless, examples of which include bleaching, deodorization, air purification, the removal of colloids, the collection of gases and solvents, the improvement of crystalline properties, the stabilization of product properties and the preparation of flavoring agents. Active carbon is made by carbonizing, or thermally decomposing, vegetable matter such as wood and fruit rinds, mineral matter such as coal and fossil fuels, and waste matter such as the wastewater from pulp plants. Numerous methods are available for producing active carbon, depending on the intended type of active carbon material, the method of activation, the perforation method and other conditions. Basically, the primary material is carbonized or thermally decomposed at a temperature of 700°C or above, and is then oxidized by being mixed with steam, carbon dioxide or air at temperatures of around 900°C to 1000°C. The result is porous carbon having a very active surface area.

Active carbon, as mentioned above, is available in powder form (100 meshes or less) or granular/crushed form (5 to 30 meshes). Depending on the usage, active carbon is produced in varying shapes and forms, based on the different materials used, its granular density, void percentage, surface area, average pore diameter and so on.

Technologies for the production of active carbon are discussed in various literature, including *Kasseitan Dokuhon* (Textbook on Active Carbon) by Nobuo Ishizaki and Hiroshi Yanai, pp.108 to 156, Nikkan Kogyo Shimbun; *Shinpan Kasseitan: Kiso to Oyo* (Active Carbon: Basics and Application - New Edition) by Yuzo Sanada and Motoyuki Suzuki, pp.54 to 70, Kodansha Scientific; and *Kasseitan no Hanashi* (Story of Active Carbon) by Hideki Tatemoto, pp. 85 to 97, Nikkan Kogyo Shimbun. The present invention improves on these known production technologies in regard to the production of active carbon.

### Summary of the Invention

The present invention aims to utilize the wide-ranging benefits of active carbon, particularly in the fields of bioactive materials and health/medical products. The present invention strives to supply bioactive water in which ultra-fine active carbon particles are dispersed, by burning the active carbon powder pre-dispersed in high-pressure water through the combustion of a gaseous mixture of hydrogen and oxygen, thus facilitating the efficient production of bioactive water containing dispersed active carbon powder, which in turn provides health and medical benefits by means of bioactivity.

The high-function water obtained through the present invention provides high bioactivity and is therefore suitable for use in health products, medical products and cosmetics.

In the context of the present invention, water in which ultra-fine particles of burned active carbon powder are micro-dispersed is called "micro-dispersed active carbon water."

The micro-dispersed active carbon water obtained through the present invention is a new creation not heretofore available for production. The present invention provides a new method and system for producing water in which ultra-fine active carbon particles are micro-dispersed, where said dispersion is achieved using the heat generated through the combustion of a gaseous mixture of oxygen and hydrogen.

In other words, after studying ways to efficiently and economically produce micro-dispersed active carbon water and utilize it for purposes of bioactivity improvement, the inventors came up with the idea of combusting hydrogen and oxygen and then heating and burning active carbon powder in the combustion gas, and decided to use high-pressure water as the medium for said hydrogen and oxygen combustion in order to prevent the generation of byproducts other than water and ultra-fine active carbon particles.

Regarding conventional active carbons or active carbon oxides obtained via the simple burning of active carbon, (burned) active carbon powder particles are dispersed only temporarily in water and will quickly separate from the solution and deposit. On the other hand, in the micro-dispersed active carbon water obtained through the present invention, which is primarily characterized by burning fine active carbon particles in high-pressure water using combustion heat, the active carbon particles will not separate or deposit and will remain in stable condition for a relative long period of time.

The micro-dispersed active carbon water thus obtained also exhibits an unexpected yet remarkable bioactivity, which is achieved via the interaction of water molecules and ultra-fine active carbon particles.

The micro-dispersed active carbon water obtained through the present invention is considered suitable for the above purpose at the present. Moreover, it is sure to provide an innovative bioactive material that will duly meet the needs of today's health-conscious consumers. Although the mechanism is not yet clear as to why water containing dispersed ultra-fine active carbon particles exhibits such bioactivity, the inventors are working diligently to find chemical explanations for this effect.

The basic feature of the present invention lies in the dispersion of active carbon powder in high-pressure water using the heat generated via the combustion of a gaseous mixture of oxygen and hydrogen, so as to produce bioactive water in which fine particles of active carbon powder are dispersed. Specifically, the invention consists of the elements specified in items 1 through 5 below:
(1) A method for producing bioactive water in which ultra-fine active carbon particles are dispersed, by dispersing active carbon particles in high-pressure water and then combusting a gaseous mixture of oxygen' and hydrogen in said high-pressure water to burn the active carbon particles using the resultant combustion gas.
(2) A system for producing bioactive water in which ultra-fine active carbon particles are dispersed, comprising a high-pressure water storage tank, a nozzle for injecting a gaseous mixture of oxygen and hydrogen, an active carbon powder rod, and a pressure-resistant container with an ignition device and combustion chamber.
(3) The system specified in item 2 above for producing bioactive water in which ultra-fine active carbon particles are dispersed, being further equipped with a water electrolyzer for generating oxygen and hydrogen gases and/or a filter system.
(4) Health products, medical products or cosmetics containing the ultra-fine active carbon particles dispersed in high-pressure water being produced by the method specified in item 1 above.
(5) Health products, medical products or cosmetics containing the ultra-fine active carbon particles dispersed in high-pressure water being produced by the system specified in item 2 or 3 above.

### Brief Description of the Drawings

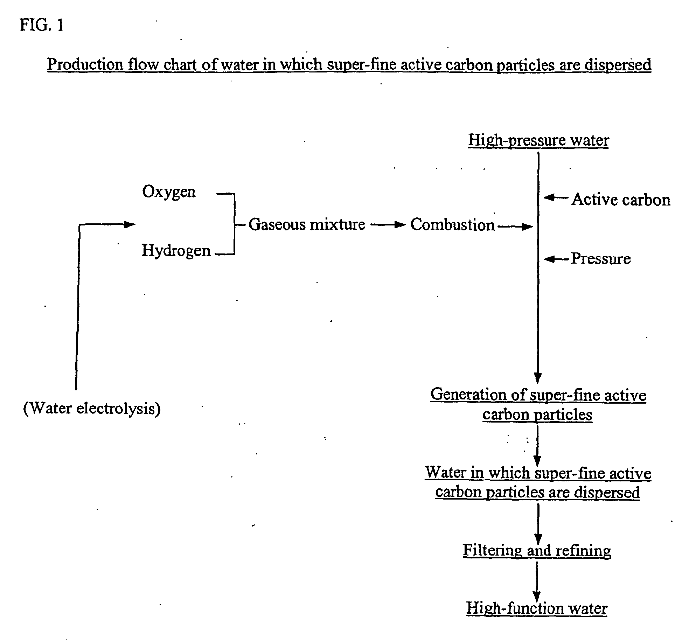

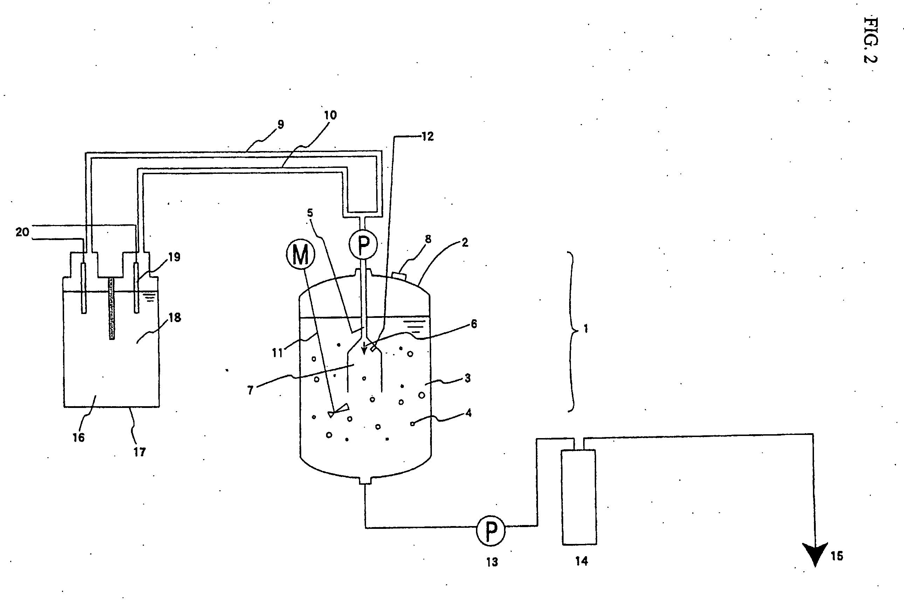

FIG. 1 shows a production flow chart of dispersed active carbon water as obtained through the present invention, while FIG. 2 provides a schematic drawing of the production system for dispersed active carbon water as given in the present invention.

The legend for the drawing is described below:
1: Production system for dispersed active carbon water as given in the present invention
2: Pressure-resistant container
3: High-pressure water
4: Active carbon
5: Mixture gas channel
6: Mixture-gas injection port
7: Combustion area
8: Pressure-regulating valve
9: Hydrogen supply channel
10. Oxygen supply channel
11: Agitator
12: Ignition device
13: Pump
14: Filter system
15: Product output port
16: Water
17: Water electrolyzer
20: Electrode

### Best Mode for Carrying Out the Invention

The active carbon material used in the present invention may be active carbon powder or active carbon in granular or crushed form, as explained earlier. However, the powder type is more suitable in terms of combustion efficiency. Additionally, in light of the usage, only those active carbons used in the production of refined sugar, amino acids, starch, isomerized sugar, medicines, brewed foods and liquors, oils and fats and other food/drink-related products should be used. In the method given by the present invention, the input volumes of combusting fuel gases, being gaseous hydrogen and oxygen, the reactive pressure within the combustion chamber, the pressure of high-pressure water and the input volume of active carbon must all be controlled. Additionally, after the active carbon is burned the water will naturally contain a small volume of oxidized active carbon in addition to pure ultra-fine active carbon particles, and these oxidized active carbon particles must be filtered as necessary. The ideal internal pressure of the tank is approximately 2 to 3.5 atmospheres, and the input volume of the gaseous hydrogen/oxygen mixture should be approximately 5 liters per second.

Regarding the present invention, the method and system for producing dispersed active carbon water in which fine active carbon particles are dispersed in high-pressure water, as specified above, are explained using the drawings.

The dispersed active carbon water as given in the present invention is produced through the process specified by the flow chart in FIG. 1. Specifically, micro-dispersed active carbon water is produced using the system illustrated in FIG. 2.

Namely, the production system (1) for micro-dispersed active carbon water as given in the present invention, and as illustrated in FIG. 2, consists of a water electrolyzer (17) for generating material gases, a pressure-resistant container (2) for producing water in which ultra-fine active carbon particles are dispersed, and a filter system (14) for filtering the micro-dispersed active carbon water. The water electrolyzer (17) consists of regular water (16) and electrodes (20), and is connected to supply channels (9, 10) used for supplying hydrogen and oxygen to the pressure-resistant container (2).

The pressure-resistant container (2) is the base system for producing bioactive water in which fine active carbon particles are dispersed, being a tank made of metal, ideally steel, as suitable for storing high-pressure water. It contains high-pressure water (3) and active carbon (4), and has a device for combustion of the gaseous hydrogen/oxygen mixture, a pressure-regulating valve (8) and an agitator (11). The combustion device consists of a channel (5) for the gaseous hydrogen/oxygen mixture, a mixture-gas injection port (6) and a mixture-gas combustion area (7).

The pressure-resistant container is filled with high-pressure water (3) in which active carbon powder (4) is pre-dispersed. The pressure-resistant container takes in the hydrogen and oxygen supplied via the hydrogen supply channel (9) and oxygen supply channel (10) that feed the respective material gases generated in the water electrolyzer (17), and then discharges a gaseous mixture via the injection port (6) into the combustion area (7) under high pressure. The gaseous mixture of hydrogen and oxygen is then ignited by an ignition device (12), and ultra-fine particles of active carbon powder being burned by the gaseous mixture are released into high-pressure water (3). The high-pressure water (3) containing these particles is removed via the output port provided at the bottom of the high-pressure water storage tank, travels through a pump (13), and is filtered by a filter system (14) to become the final product.

In this system the water electrolyzer (17) used to generate the material gases may be replaced by high-pressure cylinders containing hydrogen and oxygen gases, respectively. However, with the present invention a process of water electrolysis is used to supply pure oxygen and hydrogen gases, thereby ensuring the efficient supply of material fuel gases.

In the system given with the present invention, a gaseous mixture of hydrogen (9) and oxygen (10) generated via electrolysis travels through the channel (5) via the pump and is injected from the injection port (6) into the combustion area (7), where the mixture gas is completely combusted to generate ultra-high-temperature steam. The heat generated by this combustion gas is then used to heat and burn active carbon powder. As for the combustion of fuel gases, the mixture ratio of hydrogen (9) and oxygen (10) must be controlled at a precise ratio of 2:1. Additionally, a tank internal-pressure regulating valve (8) must be provided to adjust the pressure within the high-pressure water storage tank.

The active carbon (4) that is heated and burned inside the combustion area (7) of the combustion device is released into high-pressure water (3) from the combustion area (7). The ultra-fine active carbon particles are thus generated in the water, after which the particles having a strong hydrophobic property are dispersed in water in stable condition.

According to the production method as given in the present invention, to produce one ton of dispersed active carbon water, for example, 1 to 10 kilograms of active carbon should be pre-dispersed in 1000 liters of water and a mixture gas pressurized at 2 to 5 atmospheres should be injected into the high-pressure storage tank set to an internal pressure of 1.5 to 5 (atmospheres) at a speed of approximately 1 to 8 liters per second. If the gas pressure is too high, the system's structure may be damaged. If the gas pressure is low, the gas may be blown upward from the combustion chamber. In this case, the heated/burned fine active carbon particles could be directly enveloped in air bubbles and diffused out of the water, thereby diminishing the production efficiency of ultra-fine active carbon particles.

In this system, hydrogen and oxygen must be combusted in water at high temperature to prevent the generation of byproducts other than fine powder or ultra-fine particles of active carbon. At this time, it is particularly important that the combustion be made to happen under high pressure, so that the hydrogen and oxygen will combust in water in a manner free of impurities. The two must be combusted at a level where the mixture gas achieves complete combustion and fully becomes ultra-high-temperature steam.

Illustrated below are sample operating conditions for the aforementioned production system, as given in the present invention for the production of one ton of dispersed active carbon water:

### Application Conditions (example)

Internal pressure of production tank: 2 atmospheres
Mixture gas: 4 atmospheres
Mixture-gas injection speed: 5 liters per second
Input volume of active carbon powder: 5 kilograms per 1000 liters of water

Bioactive water in which ultra-fine active carbon particles are dispersed was obtained under the above conditions.

If used as drinking water, the produced dispersed active carbon water must be filtered (by causing the water to sequentially travel through filters consisting of hollow-thread membranes of 50, 25, 3, 0.5 and 0.1 microns in size).

Another feature of the present invention is the use, after appropriate refining, of dispersed active carbon water produced using the above-described method in which ultra-fine active carbon particles are dispersed, as a material for health products, cosmetics, foods, medicines and quasi-medicines. However, the produced water contains a small volume of oxidized active carbon powder generated via oxygen binding, and thus requires filtering and refining.

To facilitate the filtering of the water thus obtained, it is recommended that a filter system be used so as not to remove more of the generated ultra-fine particles of active carbon powder than is necessary. In other words, selecting a filter appropriate for the required grain size facilitates the production of dispersed active carbon water suitable for the intended purpose, so base water meeting the relevant standards with regard to food sanitation, cosmetics, medicines and so on can be produced.

### Industrial Field of Application

The present invention provides a source of bioactive water by utilizing a new method and system for producing micro-dispersed active carbon water of original formulation, and by harnessing the bioactivity of said dispersed active carbon water, and facilitates the efficient production of water in which ultra-fine active carbon particles are dispersed. A number of monitor surveys suggest that the dispersed active carbon water thus obtained can be used in the production of health products, medical products; cosmetics and so on.

## Claims

1. A method for producing bioactive water in which ultra-fine active carbon particles are dispersed, comprising dispersing active carbon powder in high-pressure water and then combusting a gaseous mixture of oxygen and hydrogen in said high-pressure water to burn said active carbon powder using the resultant combustion gas.

2. An apparatus for manufacturing bioactive water in which ultra-fine active carbon particles are dispersed, comprising a high-pressure water storage tank, a nozzle for injecting a gaseous mixture of oxygen and hydrogen, an active carbon powder rod, and a pressure-resistant container with an ignition device and combustion chamber.

3. The apparatus for manufacturing bioactive water as described in claim 2, further comprising a water electrolyzer for generating oxygen and hydrogen gases and/or a filter.

4. Health products, medical products or cosmetics containing the ultra-fine active carbon particles dispersed in high-pressure water as produced using the method described in claim 1.

5. Health products, medical products or cosmetics containing the ultra-fine active carbon particles dispersed in high-pressure water as produced using the apparatus described in claim 2 or 3.
